# FASCICULE DE BREVET EUROPEEN

(11) **EP 0 560 652 B1**
(45) Date de publication et mention de la délivrance du brevet: **06.08.1997**
(21) Numéro de dépôt: 93400561.2
(22) Date de dépôt: 04.03.1993
(51) Int. Cl.: A61F 2/64

(54) **Dispositif de régulation de la marche des amputés fémoraux**
Steuereinrichtung für Oberschenkelprothese
Control device for above knee prosthesis

(30) Priorité: 11.03.1992 FR 9202901
(43) Date de publication de la demande: 15.09.1993
(73) Titulaire: ETABLISSEMENTS PROTEOR Société anonyme dite:, 21100 Dijon (FR)
(72) Inventeur: Bouchard, Jean-Claude, F-39500 Tavaux (FR); Vera, Bernard, F-21250 Seurre (FR)
(74) Mandataire: Jolly, Jean-Pierre

(56) Documents cités:
- EP-A- 0 097 226
- DE-C- 724 959
- DE-C- 1 075 277
- FR-A- 1 565 589
- US-A- 3 316 558

## Description

La présente invention concerne un dispositif de régulation hydraulique de la marche des amputés fémoraux.

On sait que le moignon de ces amputés est logé dans un élément de forme complémentaire, dit "emboîture", articulé lui-même sur un élément de jambe artificielle. Ces deux organes sont en outre réunis, généralement, par un amortisseur hydraulique, qui a une fonction de régulation de la marche de la personne amputée. Dans ce but, une tige d'un pis ton de l'amortisseur est articulée sur l'emboîture, tandis que le corps cylindrique de l'amortisseur est articulé sur l'élément de jambe (ou inversement), et la circulation du fluide hydraulique dans cet amortisseur est conçue de manière à apporter une assistance à la personne appareillée, tant dans la phase d'extension que dans la phase de flexion de la marche, en opposant notamment une forte résistance à la flexion dans toutes les situations de marche, en particulier en cas de faux pas.

FR-A-1 565 589 décrit par exemple une articulation de prothèse pourvue d'un dispositif de rappel hydraulique à amortissement prépondérant dans un sens et blocage pour une vitesse de mouvement supérieure à une valeur prédéterminée. Cette articulation comprend un cylindre actif contenant un piston déplacé au cours du mouvement de l'articulation et un accumulateur d'énergie élastique, liés entre eux par des communications, dont une au moins comporte un clapet anti-retour, alors qu'une autre au moins présente un clapet entraîné dynamiquement à l'obturation sur son siège par un mouvement de fluide hydraulique à une vitesse supérieure à une valeur prédéterminée.

C'est à ce type de dispositif de régulation hydraulique de la marche que s'intéresse la présente invention et elle vise plus précisément à apporter un freinage constant et contrôlé en cas de chute de la personne appareillée.

Un premier but de l'invention est donc de proposer un dispositif de la régulation de la marche des amputés fémoraux qui oppose un freinage constant et contrôlé à la chute éventuelle du patient.

Un autre but de l'invention est de proposer un dispositif de régulation hydraulique de ce type, dans lequel la pression du fluide hydraulique, à partir de laquelle le freinage intervient, peut être réglée en tenant compte des capacités physiques de la personne appareillée.

A cet effet, l'invention a pour objet un dispositif de régulation hydraulique de la marche des amputés fémoraux, du type des amortisseurs hydrauliques, comprenant un premier piston se déplaçant dans un cylindre en entraînant un fluide hydraulique, un premier circuit de circulation de ce fluide hydraudique débouchant par au moins un premier orifice dans une partie plane d'une extrémité du cylindre et communiquant avec l'extrémité opposée de ce cylindre, un réservoir de fluide hydraulique sous pression communiquant avec ce premier circuit, ce réservoir comprenant une paroi mobile constituant un second piston, sollicitée par un moyen élastique de rappel, le premier pis ton étant destiné à être articulé sur une emboîture coiffant le moignon d'un amputé, tandis que le corps du cylindre peut être articulé sur un élément de jambe artificielle (ou inversement), l'élément de jambe étant lui-même articulé sur l'emboîture, ce dispositif comprenant en outre, une soupape, sollicitée par le fluide hydraulique à l'encontre d'un moyen élastique de rappel lorsque la pression du fluide hydraulique, sous la sollicitation du premier piston, atteint un seuil prédéterminé, à la suite d'un mouvement involontaire de l'amputé, ce dispositif étant caractérisé en ce que la soupape est apte à obturer le premier orifice du premier circuit hydraulique, en ce qu'un second circuit hydraulique partiellement indépendant du premier comprend un conduit, constituant un conduit de fuite contrôlée, qui débouche au-dessous de la soupape, et en ce qu'un moyen de commande est prévu pour déplacer la soupape de manière à amener ou non en regard une lumière de cette soupape et l'orifice du conduit correspondant de fenêtre contrôlée.

Le moyen de commande de la soupape pourra avantageusement entraîner celle-ci en rotation.

De préférence, le moyen de rappel élastique sollicitant le clapet sera réglable, de manière à pouvoir adapter le dispositif au degré d'autonomie de l'amputé.

De préférence, également, un système commandable de blocage de la soupape en position d'obturation du premier orifice sera prévu, de manière à permettre à l'amputé de bloquer volontairement cette soupape, en obturant ou non le second orifice de passage contrôlé du fluide hydraulique. Lorsque ce second orifice sera laissé libre, un mouvement de freinage sera opposé aux déplacements du piston et ceci pourra être utile pour l'amputé, par exemple, dans le cas d'une descente d'escalier. Lorsque le premier et le second orifices seront tous deux obturés, le piston sera bloqué en position dans la phase de compression.

Les dessins schématiques annexés, qui n'ont pas de caractère limitatif, illustrent une forme de réalisation de l'invention. Toutes les figures des dessins, à l'exception de la figure 6, sont des vues en coupe axiale du dispositif, dans des phases d'utilisation différentes, à savoir :
- Figure 1 : pendant la phase de traction du piston du dispositif de régulation hydraulique, au cours d'une marche normale de l'amputé ;
- Figure 2 : pendant la phase de compression du piston du dispositif, également au cours d'une marche normale de l'amputé ;

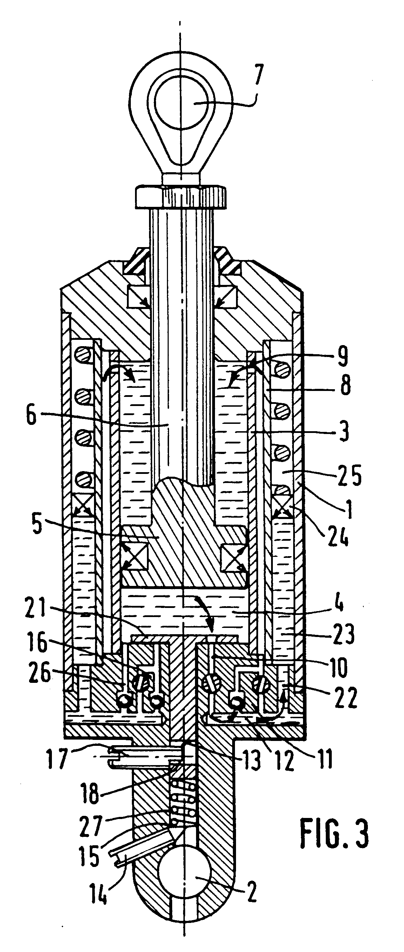

- Figure 3 : dans le cas d'une perte d'équilibre de l'amputé, à la suite d'un mouvement involontaire de celui-ci ;
- Figure 4 : après blocage partiel volontaire du dispositif, par exemple pour assister l'amputé se déplaçant dans un escalier ;
- Figure 5 : après blocage volontaire complet du dispositif ;
- La figure 6 est une vue partielle de dessus de la soupape du dispositif, dans la position des figures 1,2 et 3 ;
- La figure 7 est une vue analogue à la figure 6, lorsque le dispositif est dans la position de la figure 4;

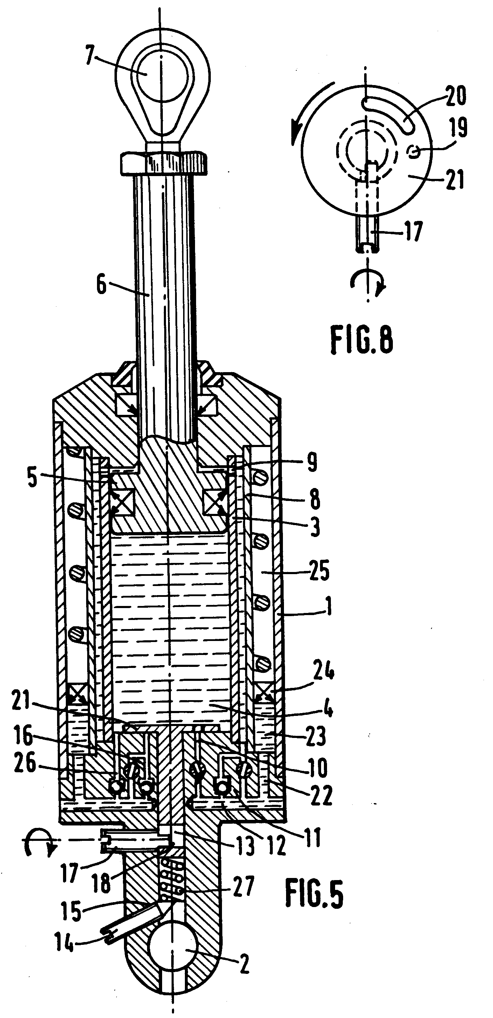

- La figure 8 est une vue analogue aux figures 6 et 7, dans la position occupée par le dispositif sur la figure 5.

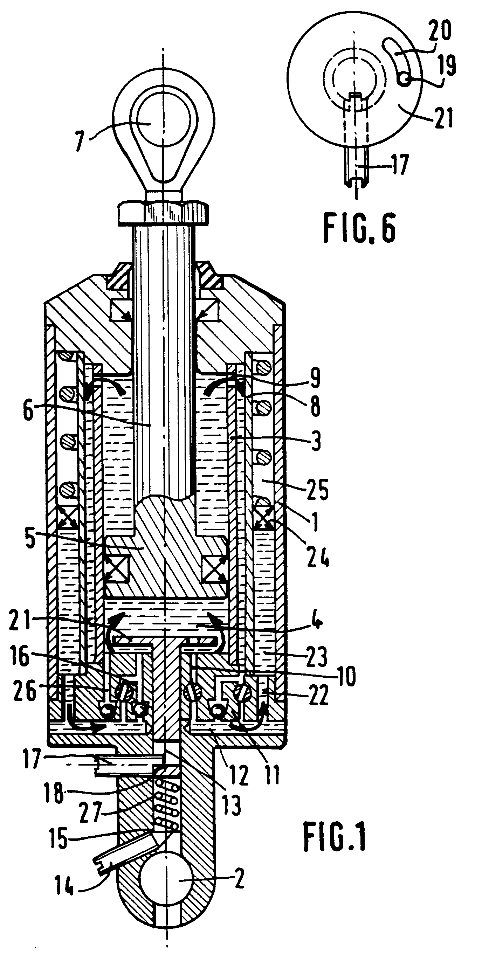

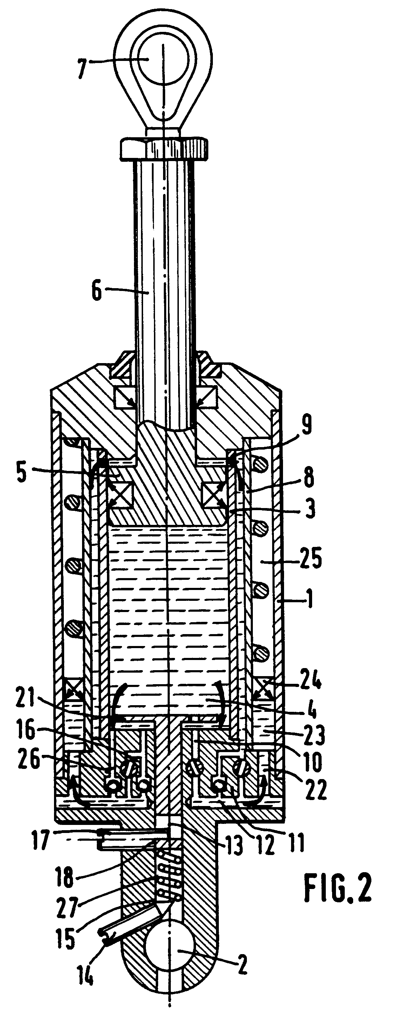

On se référera d'abord aux figures 1 et 2.

Comme on le voit, le dispositif représenté comprend un corps d'amortisseur 1, dont une extrémité est destinée à être articulée par un axe 2 sur la jambe artificielle (non représentée) d'un amputé fémoral. Dans la partie centrale de ce dispositif est ménagé un premier cylindre 3 contenant un fluide hydraulique 4 et dans lequel se déplace un piston 5, dont la tige 6 fait saillie en dehors du corps 1 et est articulée à son extrémité par un axe 7 sur l'emboîture (non représentée) dans laquelle est logé le moignon de l'amputé. Une disposition inverse pourrait naturellement être adoptée, avec le corps 1 articulé sur l'emboîteur et la tige 6 articulée sur la jambe artificielle. L'emboîture et la jambe artificielle sont naturellement articulées entre elles de façon usuelle.

Un second cylindre 8, à section annulaire, est disposé concentriquement au cylindre 3 et peut communiquer avec celui-ci, d'une part, à sa partie supérieure, par des orifices 9 ménagés dans sa paroi latérale, d'autre part, à sa partie inférieure, par des conduits 10,26,16 débouchant dans le fond du cylindre 3, par un collecteur 12 et par un conduit 11, débouchant dans le fond du cylindre 8.

Une soupape 21, sollicitée par le fluide hydraulique 4, à l'encontre d'un ressort de rappel 27, qui sollicite sa tige 13, permet d'obturer éventuellement les orifices par lesquels les conduits 16,10 débouchent dans le fond du cylindre 3. Une vis 14, commandable de l'extérieur du dispositif, permet de déplacer un disque mobile 15 sur lequel prend appui le ressort 27, en vue de régler la tension de celui-ci et la pression qu'il exerce sur la soupape 21.

La soupape 21 peut être entraînée en rotation par un ergot mobile en rotation ou excentrique 17, qui est engagé dans un évidement 18 de la tige 13 de la soupape et qui fait saillie latéralement à l'extérieur du dispositif.

Un conduit 10 de fuite contrôlée, communiquant avec le collecteur 12, débouche dans le fond du cylindre 3 par un orifice 19 (voir figure 6), à l'aplomb duquel peut être amenée une lumière 20, en forme d'arc de cercle, ménagée dans la soupape, de sorte que, en fonction de la position de la soupape 21, le conduit 10 et par conséquent le collecteur 12 peuvent communiquer ou non avec le cylindre 3, même lorsque la soupape est appliquée contre le fond du cylindre.

Le collecteur 12 communique enfin par des conduits 22 avec un réservoir cylindrique 23, à section circulaire, disposé concentriquement au cylindre 8, dont la partie supérieure est constituée par un élément annulaire mobile 24, formant piston, sollicité par un ressort 25, qui, à son autre extrémité, prend appui contre la partie supérieure du corps 1.

Le fonctionnement de ce dispositif est le suivant.

En position normale de marche d'une personne amputée du fémur, dont le moignon est logé dans une emboîture articulée sur une jambe artificielle, avec un dispositif de régulation hydraulique conforme à l'invention interposé entre l'emboîture et la jambe, la soupape 21 ne repose pas sur le fond du cylindre 3 (figures 1 et 2). Le fluide hydraulique, sous la sollicitation du piston 5, circule donc normalement, de façon connue en soi, dans le sens indiqué par les flèches, entre les cylindres 3 et 8, de part et d'autre du piston 5, et entre ces cylindres et le réservoir 23, aussi bien à la phase de compression du fluide hydraulique dans le cylindre 3 (figure 2) qu'à la phase de traction du piston 5 (figure 1). Comme indiqué ci-dessus, une partie du fluide hydraulique emprunte le conduit 10 de fuite contrôlée.

Si, à la suite d'un faux mouvement de la personne amputée, d'une chute de celle-ci par exemple, la vitesse de déplacement du piston 5 devient importante, la soupape 21, sous l'effet de la pression et de la vitesse du fluide, vient se plaquer contre le fond du cylindre 3, en obturant l'orifice des conduits 16. L'orifice 19 du conduit de fuite contrôlée 10 est cependant en coïncidence avec la lumière 20 de la soupape, et le fluide hydraulique peut donc continuer à emprunter le conduit 10 en freinant ainsi la descente du piston 5. La pression à partir de laquelle la soupape 21 vient se plaquer contre le fond du cylindre 3 peut être réglée et adaptée aisément au degré d'autonomie de la personne amputée en agissant sur la vis de réglage 14.

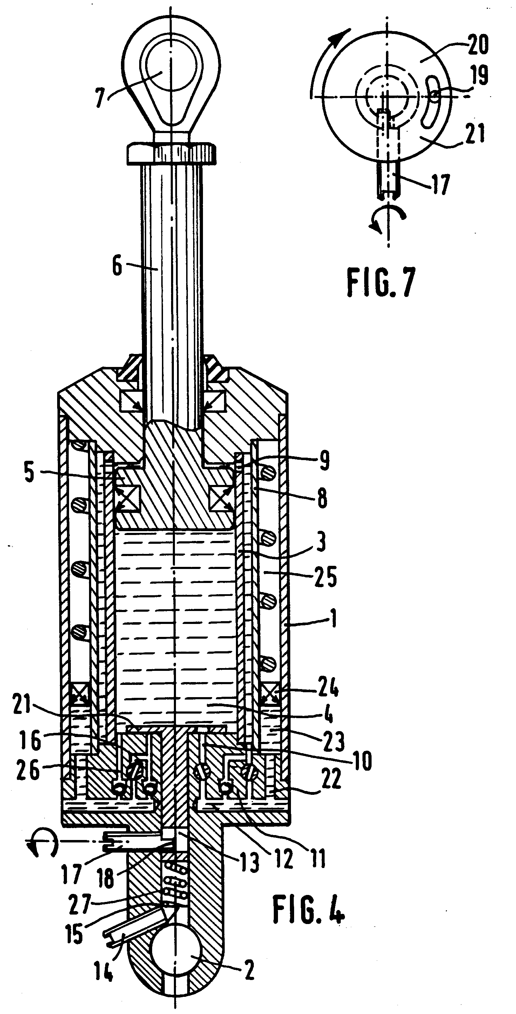

Si, pour exercer certains mouvements, cette personne désire bloquer partiellement le dispositif, par exemple pour descendre un escalier, en ne laissant accessible au fluide hydraulique que le conduit de fuite contrôlée 10 (figure 4), il lui suffira d'actionner l'excentrique 17 dans un sens approprié, par exemple dans le sens des aiguilles d'une montre, pour faire descendre la soupape 21 et la faire tourner simultanément, mais en laissant l'orifice 19 du conduit 10 en communication avec la lumière 20 de la soupape.

Enfin, dans le cas où la personne amputée veut bloquer complètement le dispositif (figure 5), elle actionnera l'excentrique 17 en sens inverse du cas précédent (cas de la figure 4), pour abaisser la soupape 21 et l'amener à obturer simultanément l'orifice 19 du conduit de fuite contrôlée 10 (figure 6), la phase d'extension restant toujours possible grâce au conduit 25.

Le dispositif conforme à l'invention apporte donc, sous une forme simple, des perfectionnements importants aux dispositifs de régulation hydraulique de la marche des amputés fémoraux.

## Revendications

1. Dispositif de régulation hydraulique de la marche des amputés fémoraux, du type des amortisseurs hydrauliques, comprenant un premier piston (5) se déplaçant dans un cylindre (3) en entraînant un fluide hydraulique, un premier circuit (8, 9, 10, 11,16) de circulation de ce fluide hydraudique débouchant par au moins un premier orifice dans une partie plane d'une extrémité du cylindre (3) et communiquant avec l'extrémité opposée de ce cylindre, un réservoir (23) de fluide hydraulique sous pression communiquant avec ce premier circuit, ce réservoir comprenant une paroi mobile (24) constituant un second piston, sollicitée par un moyen élastique de rappel (25), le premier piston (5) étant destiné à être articulé sur une emboîture coiffant le moignon d'un amputé, tandis que le corps (1) du cylindre peut être articulé sur un élément de jambe artificielle (ou inversement), cet élément de jambe étant lui-même articulé sur l'emboîture, ce dispositif comprenant en outre une soupape (21), sollicitée par le fluide hydraulique à l'encontre d'un moyen élastique de rappel (27) lorsque la pression du fluide hydraulique, sous la sollicitation du premier piston (5), atteint un seuil prédéterminé, à la suite d'un mouvement involontaire de l'amputé, ce dispositif étant caractérisé en ce que la soupape (21) est apte à obturer le premier orifice du premier circuit hydraulique, en ce qu'un second circuit hydraulique partiellement indépendant du premier, comprend un conduit (10) constituant un conduit de fuite contrôlée qui débouche au-dessous de la soupape (21) et en ce qu'un moyen de commande (17) est prévu pour déplacer la soupape (21) de manière à amener ou non en regard une lumière (20) de cette soupape et l'orifice (19) correspondant du conduit (10) de fuite contrôlée.

2. Dispositif selon la revendication 1, caractérisé en ce que le circuit emprunté par le fluide hydraulique pour passer de part et d'autre du piston comprend un second cylindre annulaire (8) concentrique au premier cylindre (3).

3. Dispositif selon l'une des revendications 1 et 2, caractérisé en ce que le réservoir (23) a une section annulaire et est concentrique au cylindre (3).

4. Dispositif selon l'une des revendications 1 à 3, caractérisé en ce que le moyen de commande (17) entraîne la soupape (21) en rotation.

5. Dispositif selon la revendication 4, caractérisé en ce que le moyen de commande (17) de la soupape (21) permet d'appliquer celle-ci contre les orifices des premier et second circuits hydrauliques.

6. Dispositif selon l'une des revendications 1 à 5, caractérisé en ce que la tension du moyen de rappel élastique (27) de la soupape (21) est réglable.

## Claims

1. A device for hydraulically controlling the walking of femoral amputees, of the hydraulic shock absorber type, comprising a first piston (5) moving in a cylinder (3) and displacing a hydraulic fluid, a first circuit (8, 9, 10, 11, 16) for the circulation of this hydraulic fluid opening via at least a first orifice in a flat part of one end of the cylinder (3) and communicating with the opposite end of this cylinder, a compressed hydraulic fluid tank (23) communicating with this first circuit, this tank comprising a movable wall (24) constituting a second piston and loaded by a resilient return means (25), the first piston (5) being designed to be articulated to a casing capping the stump of an amputee, while the body (1) of the cylinder may be articulated to an artificial leg member (or vice versa), this leg member itself being articulated to the casing, this device further comprising a valve (21) loaded by the hydraulic fluid counter to a resilient return means (27) when the pressure of the hydraulic fluid, under the loading of the first piston (5), reaches a predetermined threshold, following an involuntary movement on the part of the amputee, this device being characterised in that the valve (21) is capable of sealing the first orifice of the first hydraulic circuit, in that a second hydraulic circuit, partially independent of the first, comprises a duct (10) constituting a controlled escape duct which opens below the valve (21) and in that a control means (17) is provided to move the valve (21) in such a way as to bring into position opposite each other, or not, a slot (20) in this valve and the corresponding orifice (19) of the controlled escape duct (10).

2. A device according to claim 1, characterised in that the circuit used by the hydraulic fluid to pass either side of the piston comprises a second annular cylinder (8) concentric with the first cylinder (3).

3. A device according to either one of claims 1 and 2, characterised in that the tank (23) has an annular section and is concentric with the cylinder (3).

4. A device according to any one of claims 1 to 3, characterised in that the control means (17) drives the valve (21) in rotation.

5. A device according to claim 4, characterised in that the control means (17) of the valve (21) permits the application thereof against the orifices of the first and second hydraulic circuits.

6. A device according to any one of claims 1 to 5, characterised in that the tension of the resilient return means (27) of the valve (21) is adjustable.

## Patentansprüche

1. Vorrichtung zur hydraulischen Regelung des Ganges bei Trägern von Oberschenkelprothesen in der Art hydraulischer Dämpfer, welche einen ersten Kolben (5) aufweist, der sich in einem Zylinder (3) verlagert und dabei eine Hydraulikflüssigkeit in Bewegung versetzt, sowie einen ersten Hydraulikkreis (8, 9, 10, 11, 16) für den Kreislauf dieser Hydraulikflüssigkeit, welcher über mindestens eine erste Öffnung in einen ebenen Abschnitt eines Endes des Zylinders (3) mündet und mit dem gegenüberliegenden Ende dieses Zylinders in Verbindung steht, einen Behälter (23) zur Aufnahme der unter Druck stehenden Hydraulikflüssigkeit, der mit diesem ersten Hydraulikkreis in Verbindung steht, wobei dieser Behälter eine einen zweiten Kolben bildende bewegliche Wandung (24) umfaßt, welche mittels einer elastischen Rückstelleinrichtung (25) belastet ist, und der erste Kolben (5) zur Anlenkung an einem Stumpfbett bestimmt ist, welches den Stumpf des Amputierten umgreift, wohingegen das Hauptteil (1) des Zylinders an einem künstlichen Schenkelteil (bzw. umgekehrt) anlenkbar ist, und dieses Schenkelteil seinerseits an dem Stumpfbett angelenkt ist, und wobei diese Vorrichtung des weiteren ein Ventil (21) aufweist, das von der Hydraulikflüssigkeit entgegen einer elastischen Rückstelleinrichtung (27) beaufschlagt wird, wenn der Druck der Hydraulikflüssigkeit unter Beaufschlagung durch den ersten Kolben (5) im Anschluß an eine ungewollte Bewegung des Amputierten einen vorgegebenen Schwellwert erreicht, **dadurch gekennzeichnet,** daß das Ventil (21) zum Verschließen der ersten Öffnung im ersten Hydraulikkreis ausgelegt ist, daß ein vom ersten Hydraulikkreis teilweise unabhängiger zweiter Hydraulikkreis eine Leitung (10) umfaßt, welche eine Leckleitung mit kontrollierter Ableitung bildet, die unterhalb des Ventils (21) mündet, und daß zur Bewegung des Ventils (21) eine Betätigungseinrichtung (17) in der Weise vorgesehen ist, daß sie gegebenenfalls ein Langloch (20) dieses Ventils und die entsprechende Öffnung (19) der Leckleitung (10) mit kontrollierter Ableitung in gegenseitige Gegenüberstellung bringt.

2. Vorrichtung nach Anspruch 1, **dadurch gekennzeichnet**, daß der von der Hydraulikflüssigkeit zum Durchtritt von der einen Seite des Kolbens zur anderen verwendete Hydraulikkreis einen zweiten, zum ersten Zylinder (3) konzentrischen ringförmigen Zylinder (8) umfaßt.

3. Vorrichtung nach einem der Ansprüche 1 und 2, **dadurch gekennzeichnet,** daß der Behälter (23) einen ringförmigen Querschnitt aufweist und zum Zylinder (3) konzentrisch angeordnet ist.

4. Vorrichtung nach einem der Ansprüche 1 bis 3 **dadurch gekennzeichnet**, daß die Betätigungseinrichtung (17) das Ventil (21) in Drehbewegung versetzt.

5. Vorrichtung nach einem der Ansprüche 1 und 2, **dadurch gekennzeichnet,** daß die Betätigungseinrichtung (17) für das Ventil (21) dessen Anlage gegen die Öffnungen des ersten und des zweiten Hydraulikkreises gestattet.

6. Vorrichtung nach einem der Ansprüche 1 und 2, **dadurch gekennzeichnet,** daß die Spannung der elastischen Rückstelleinrichtung (27) für das Ventil (21) einstellbar ist.
